(19) Europäisches Patentamt
European Patent Office
Office européen des brevets

(11) Veröffentlichungsnummer: **0 073 973**
**A1**

# (12) EUROPÄISCHE PATENTANMELDUNG

(21) Anmeldenummer: 82107510.8

(22) Anmeldetag: 18.08.82

(51) Int. Cl.³: **C 07 D 275/02**
C 07 D 277/56, C 07 D 277/58
C 07 D 417/12, A 01 N 43/78
A 01 N 43/80

(30) Priorität: 24.08.81 DE 3133418

(43) Veröffentlichungstag der Anmeldung: 16.03.83 Patentblatt 83/11

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Zeeh, Bernd, Dr.
Thorwaldsenstrasse 5
D-6700 Ludwigshafen(DE)

(72) Erfinder: Rentzea, Costin, Dr.
Neuenheimer Landstrasse 72
D-6900 Heidelberg(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

## (54) Thiazolyl- und Isothiazolylcarbonsäureanilide, Verfahren zu ihrer Herstellung und ihre Verwendung als Fungizide.

(57) Thiazolyl- und Isothiazolylcarbonsäureanilide der Formel I

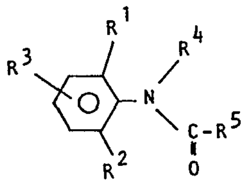

I,

worin
$R^1$ Alkyl, Alkoxy oder Halogen, $R^2$ Alkyl, Alkoxy, Halogen oder $NO_2$, $R^3$ Wasserstoff oder Alkoxy,

$R^4$ 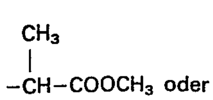 oder 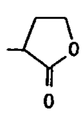 und

$R^5$ einen gegebenenfalls substituierten 3- oder 4- oder 5-Isothiazolyl- oder 2- oder 4- oder 5-Thiazolylrest bedeutet und diese enthaltende Fungizide.

EP 0 073 973 A1

Croydon Printing Company Ltd.

# Thiazolyl- und Isothiazolylcarbonsäureanilide, Verfahren zu ihrer Herstellung und ihre Verwendung als Fungizide

Die vorliegende Erfindung betrifft neue Thiazolyl- und Isothiazolylcarbonsäureanilide, Verfahren zu ihrer Herstellung, Fungizide, die diese Verbindungen als Wirkstoffe enthalten, sowie Verfahren zur Bekämpfung von Pilzen.

Heterocyclische Carbonsäureanilide, die fungizid wirksam sind, sind aus der DE-OS 25 13 732 bekannt. Heterocyclische Carbonsäurereste in diesen Verbindungen sind Carbonsäurereste, die z.B. durch Pyridyl substituiert sind. Die Wirkung dieser Verbindungen gegen Pilze aus der Klasse der Phycomyceten genügt bei niederen Konzentrationen nicht den Anforderungen der Praxis.

Es wurde nun gefunden, daß Thiazolyl- und Isothiazolylcarbonsäureanilide der Formel I

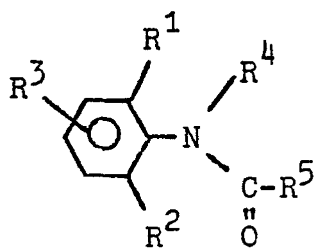

I,

in der
$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_2$-Alkoxy,

$R^4$ $-\overset{CH_3}{\overset{|}{C}H}-COOCH_3$ oder 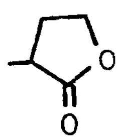 und

$R^5$ einen gegebenenfalls durch Methyl, Chlor, Brom oder Nitro substituierten 3- oder 4- oder 5-Isothiazolyl- oder 2- oder

Sws/P

4- oder 5-Thiazolylrest bedeutet, fungizid ausgezeichnet wirksam sind.

$R^1$ und $R^2$ bedeuten vorzugsweise Methyl, Ethyl, Methoxy, Ethoxy und Chlor, während $R^3$ bevorzugt Wasserstoff oder Methyl und $R^5$ bevorzugt 3-, 4- oder 5-Isothiazolyl, 3-Methyl-4-isothiazolyl, 3-Methyl-5-isothiazolyl, 5-Nitro-3-isothiazolyl oder 2-, 4- oder 5-Thiazolyl, 2-Brom-4-thiazolyl, 2-Brom-5-thiazolyl, 5-Nitro-2-thiazolyl, 4-Nitro-2-thiazolyl, 4-Methyl-5-thiazolyl oder 2-Chlor-4-methyl-5-thiazolyl bedeutet.

Die Carbonsäureanilide der Formel I besitzen ein Asymmetriezentrum in dem Rest $R^4$. Die optisch reinen Enantiomeren können nach üblichen Methoden erhalten werden. Fungizid wirksam sind sowohl die bei der Synthese üblicherweise anfallenden Gemische als auch die reinen Enantiomeren, die alle von der Erfindung umfaßt werden.

Die Thiazolyl- und Isothiazolylcarbonsäureanilide der Formel I können durch Umsetzung eines Anilinderivates der Formel II

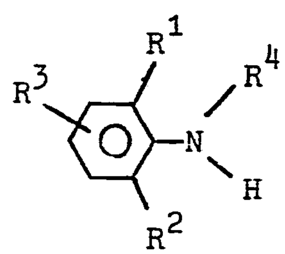

II,

in der $R^1$, $R^2$, $R^3$ und $R^4$ die oben genannten Bedeutungen haben, mit einem Carbonsäurederivat der Formel III

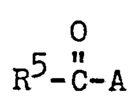

III,

in der $R^5$ die oben genannten Bedeutungen hat und A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines Reaktionsbeschleunigers bei einer Temperatur im Bereich zwischen 0 und 120°C erhalten werden.

In Formel III bedeutet A beispielsweise Halogen, wie Chlor oder Brom, Alkoxycarbonyloxyreste, wie Methoxycarbonyloxy oder Ethoxycarbonyloxy, oder den Benzyloxycarbonyloxyrest oder einen Azolylrest, wie den Imidazolyl- oder den Triazolylrest.

Die Umsetzung kann in Gegenwart eines Lösungs- oder Verdünnungsmittels durchgeführt werden. Zu den bevorzugten Lösungs- bzw. Verdünnungsmitteln gehören Halogenkohlenwasserstoffe, beispielsweise Methylenchlorid, Chloroform, 1,2-Dichlorethan, Chlorbenzol, aliphatische oder aromatische Kohlenwasserstoffe, wie Cyclohexan, Petrolether, Benzol, Toluol oder Xylole; Ester wie Essigsäureethylester; Nitrile, wie Acetonitril; Sulfoxide, wie Dimethylsulfoxid; Ketone, wie Aceton oder Methylethylketon; Ether, wie Diethylether, Tetrahydrofuran oder Dioxan oder Gemische dieser Lösungsmittel.

Zweckmäßig verwendet man das Lösungs- bzw. Verdünnungsmittel in einer Menge von 100 bis 2000 Gew.%, vorzugsweise 100 bis 1000 Gew.%, bezogen auf die Ausgangsstoffe der Formel II bzw. III.

Geeignete anorganische oder organische Basen, die gegebenenfalls als säurebindende Mittel dem Reaktionsgemisch zugesetzt werden können, sind beispielsweise Alkalicarbonate, wie Kalium- oder Natriumcarbonat; Alkalihydride, wie

Natriumhydrid oder tertiäre Amine, wie Trimethylamin, Triethylamin, N,N-Dimethylanilin, N,N-Dimethylcyclohexylamin, N-Methylpiperidin oder Pyridin; Azole, wie 1,2,4-Triazol oder Imidazol. Es können aber auch andere übliche Basen verwendet werden.

Als Reaktionsbeschleuniger kommen vorzugsweise Metallhalogenide, wie Natriumbromid oder Kaliumjodid, Azole, wie Imidazol oder 1,2,4-Triazol oder Pyridine, wie 4-Dimethylaminopyridin oder Mischungen dieser Substanzen in Betracht. Zweckmäßig setzt man auf 1 Mol Anilinderivat der Formel II 0,9 bis 1,3 Mol Säurederivat der Formel III sowie gegebenenfalls 0,5 bis 2 Mol Base und gegebenenfalls 0,01 Mol bis 0,1 Mol Reaktionsbeschleuniger ein.

Die Umsetzung wird im allgemeinen bei einer Temperatur im Bereich zwischen -10 und 100°C in einem Zeitraum von 1 bis 60 Stunden durchgeführt, drucklos oder unter Druck, kontinuierlich oder diskontinuierlich.

In einer bevorzugten Form des erfindungsgemäßen Verfahrens vermischt man den Ausgangsstoff der Formel II gegebenenfalls mit einer Base und gegebenenfalls mit einem Verdünnungsmittel, gibt dann das Säurederivat der Formel III und gegebenenfalls den Reaktionsbeschleuniger zu und hält das Reaktionsgemisch für 0,5 bis 12, vorzugsweise 1 bis 6 Stunden bei der Reaktionstemperatur, die zwischen -10 und 100°C liegen kann.

Zur Isolierung der neuen Verbindungen wird, falls vorhanden, das Verdünnungsmittel entfernt, der Rückstand wird dann in einem geeigneten Lösungsmittel gelöst und mit verdünnter Säure, dann mit wäßriger verdünnter Lauge sowie mit Wasser gewaschen, um die überschüssige Base und die Ausgangsstoffe II und III zu entfernen.

Die nach dem Abdestillieren des Lösungsmittels verbleibenden Produkte bedürfen im allgemeinen keiner weiteren Reinigung, können aber, falls erforderlich, nach bekannten Methoden, beispielsweise durch Umkristallisation, Extraktion oder Chromatographie, weiter gereinigt werden.

Die Anilinderivate der Formel II sowie Verfahren zu ihrer Herstellung sind aus der DE-OS 28 02 211, J.Org.Chem. 30, 4101-4104 (1965) und Tetrahedron 1967, 487-493 bekannt.

Die als Ausgangsstoffe eingesetzten Thiazolyl- oder Isothiazolylcarbonsäurederivate der Formel III sind teilweise bekannt bzw. können nach bekannten Methoden aus den Thiazolyl- oder Isothiazolylcarbonsäuren der Formel IV

$$R^5\text{-COOH} \qquad \text{IV}$$

hergestellt werden.

Die folgenden Tabellen geben eine Übersicht über die Carbonsäuren der Formel IV:

Tabelle I

| Ring-substituent | Stellung der COOH--Gruppe am Thiazolring | Literatur-stelle |
|---|---|---|
| H | 2 | 1 |
| 5-$NO_2$ | 2 | 2 |
| 4-$NO_2$ | 2 | 3 |
| H | 4 | 4 |
| 2-Br | 4 | 5 |
| H | 5 | 6 |
| 2-Br | 5 | 7 |
| 4-$CH_3$ | 5 | 8 |
| 2-Cl, 4-$CH_3$ | 5 | 9 |

1. H. Erlenmeyer u.a., Helv.Chim. Acta, 28, 922 (1945).
2. P. Strehlke, Eur.J.Med.Chem., 9, 35 und 41 (1974).
3. D.W. Henry, J.Med.Chem., 12, 303 (1969).
4. D.J. Tocco u.a., J.Med.Chem. 7, 399 (1964); R.A. Firestone u.a., J.Org.chem., 39, 3384 (1974).
5. H. Erlenmeyer u.a., Helv.Chim. Acta, 28, 362 (1945); R. Meyer, Chem. Heterocycl., 34, 519 (1979).
6. M. Erne, Helv.Chim. Acta, 36, 138 (1953).
7. H. Balli u.a., Helv.Chim. Acta, 59, 155 (1976).
8. P.N. Craig u.a., J.Org.Chem., 22, 559 (1957).
9. M.A. Abdel-Latef u.a., Rocz.Chem., 46, 1647 (1972); R.K. Howe, J.Org.Chem., 42, 3230 (1977).

Tabelle II

| Ring-substituent | Stellung der -COOH--Gruppe am Isothiazolring | Literatur-stelle |
|---|---|---|
| H | 3 | 10 |
| 5-$NO_2$ | 3 | 11 |
| H | 4 | 12 |
| 3-$CH_3$ | 4 | 13 |
| H | 5 | 14 |

10. A. Adams u.a., J.Chem.Soc., 3061 (1959).
11. R.J.A. Walsh u.a., J.Chem.Soc., Perkin Trans, I, 1247 (1972).
12. R. Raap u.a., J.Med.Chem. 11, 70 (1967); R. Raap u.a., U.S.A. Pat. 3 271 407.
13. A. Buttimore u.a., J.Chem.Soc., 2032 (1963).
14. J.M. Essery u.a., J.Antibiot., 27, 573 (1974).

Das folgende Beispiel erläutert die Herstellung der neuen Thiazolyl- und Isothiazolylcarbonsäureanilide der Formel I.

Beispiel 1

Zu einer Lösung von 8,3 g (0,04 Mol) N-(1-Methoxycarbonylethyl)-2',6'-dimethylanilin in 150 ml Toluol werden 5 g Natriumhydrogencarbonat zugegeben und anschließend eine Lösung von 7,3 g (0,05 Mol) Thiazolyl-4-carbonsäurechlorid in 50 ml Toluol zugetropft. Nach 12stündigem Rühren bei 25°C werden 100 ml Wasser zugegeben. Die organische Phase wird abgetrennt, zweimal mit je 50 ml Wasser gewaschen, über Natriumsulfat getrocknet und eingeengt. Der Rückstand wird bei 0°C mit 10 ml Petrolether und 5 ml Diisopropylester eine Stunde digeriert, anschließend abgesaugt, mit 20 ml Petrolether gewaschen und im Vakuum getrocknet. Man erhält 5,8 g (45,6 % d.Th.) N-(thiazol-4-yl-carbonyl)-N-(2,6-dimethylphenyl)-alanin-methylester als weiße Kristalle vom Schmelzpunkt 80 bis 82°C. (Verbindung Nr. 1)

Analog werden folgende Verbindungen hergestellt:

Tabelle III

| Verbindung | $R^1$ | $R^2$ | $R^3$ | $R^4$ | $R^5$ | Position der CO-Gruppe am Heterocyclus | Phys. Daten |
|---|---|---|---|---|---|---|---|
| 2 | $CH_3$ | $CH_3$ | H | $-\overset{CH_3}{CH}-COOCH_3$ | 5-Methyl-thiazolyl | 4 | Fp. 102°C |
| 3 | $CH_3$ | $CH_3$ | H | " | Thiazolyl | 2 | Fp. 99-100°C |
| 4 | $CH_3$ | $CH_3$ | H | " | Thiazolyl | 5 | Fp. 78-79°C |
| 5 | $CH_3$ | $CH_3$ | H | " | 2-Bromthiazolyl | 4 | Harz |
| 6 | $CH_3$ | $CH_3$ | H | " | 2-Chlor-4-Methylthiazolyl | 5 | Harz |
| 7 | $CH_3$ | $CH_3$ | 3-$CH_3$ | " | Isothiazolyl | 3 | Öl |
| 8 | $CH_3$ | $CH_3$ | H | " | Isothiazolyl | 4 | Öl |
| 9 | $CH_3$ | $CH_3$ | H | " | Isothiazolyl | 5 | Öl |
| 10 | $CH_3$ | $C_2H_5$ | H | " | Thiazolyl | 4 | Harz |
| 11 | Cl | $CH_3$ | H | " | Thiazolyl | 4 | Harz |
| 12 | $CH_3$ | $CH_3$ | H | " | 3-Methyl-isothiazolyl | 4 | Harz |
| 13 | $CH_3$ | $CH_3$ | H | 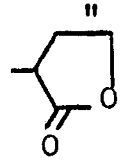 | Thiazolyl | 4 | Fp.189-190°C |
| 14 | $CH_3$ | $C_2H_5$ | H | " | Thiazolyl | 4 | Harz |
| 15 | $CH_3$ | $CH_3$ | H | " | Thiazolyl | 2 | Harz |
| 16 | $CH_3$ | $CH_3$ | H | " | Thiazolyl | 5 | Harz |
| 17 | $CH_3$ | $CH_3$ | 3-$CH_3$ | " | 5-Methyl-thiazolyl | 4 | Harz |

Tabelle III (Forts.)

| Verbindung | $R^1$ | $R^2$ | $R^3$ | $R^4$ | $R^5$ | Position der CO-Gruppe am Heterocyclus | Phys. Daten |
|---|---|---|---|---|---|---|---|
| 18 | $CH_3$ | $CH_3$ | H | 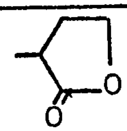 | Isothiazolyl | 3 | Öl |
| 19 | $CH_3$ | $CH_3$ | H | " | Isothiazolyl | 4 | Öl |
| 20 | $CH_3$ | $CH_3$ | H | " | Isothiazolyl | 5 | Öl |

Die neuen Wirkstoffe weisen eine starke fungitoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Die neuen Wirkstoffe zeigen eine starke fungitoxische Wirsamkeit gegen phytopathogene Pilze. Sie sind beispielsweise geeignet zur Bekämpfung von Erysiphe graminis (echter Mehltau) in Getreide, Erysiphe cichoracearum (echter Mehltau) an Kürbisgewächsen, Erysiphe polygoni an Bohnen, Podosphaera leucotricha und Phytophthora cactorum an Äpfeln,

Phytophthora infestans an Tomaten und Kartoffeln, Phytophthora parasitica an Erdbeeren, Pseudoperonospora cubensis an Gurken, Pseudoperonospora humuli an Hopfen, Peronospora destructor an Zwiebeln, Peronospora tabacina an Tabak, Peronospora sparsa an Rosen, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Uncinula necator an Reben und Spaerothaca pannosa an Rosen.

Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzenteile möglich ist.

Ferner lassen sich mit den neuen Verbindungen auch Pilze, die Keimlings- und Auflaufkrankheiten hervorrufen, bei-

spielsweise Pythium- und Aphanomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen wie Lösungen, Emulsionen, Suspensionen, Stäube, Pulver, Pasten und Granulate übergeführt werden. Die Anwendungsformen richten sich ganz nach den Verwendungszwecken; sie sollten in jedem Fall eine feine und gleichmäßige Verteilung der wirksamen Substanzen gewährleisten. Die Formulierungen werden in bekannter Weise hergestellt, z.B. durch Verstrekken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose in Betracht.

Die fungiziden Mittel enthalten 0,1 bis 95 Gew.% Wirkstoff, vorzugsweise 0,5 bis 90 Gew.% Wirkstoff.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile des Wirkstoffs Nr. 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung des Wirkstoffs Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile des Wirkstoffs Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile des Wirkstoffs Nr. 13 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile des Wirkstoffs Nr. 4 werden mit 3 Gew.--Teilen des Natriumsalzes der Diisobutylnaphthalin--alpha-sulfonsäure, 10 Gew.-Teilen des Natriumsalzes einer Ligninsulfonsäure aus einer Sulfitablauge und 67 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile des Wirkstoffs Nr. 13 werden mit 97 Gew.--Teilen feinteiligem Kaolin innig vermischt. Man erhält auf diese Weise ein Stäubemittel, das 3 Gew.-% des Wirkstoffs enthält.

VII. 30 Gew.-Teile des Wirkstoffs Nr. 3 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile des Wirkstoffs Nr. 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile des Wirkstoffs Nr. 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und anderen Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränkken.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat)
und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat)

und
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Die folgenden Versuche belegen die biologische Wirkung der neuen Verbindungen. Vergleichsmittel A ist N-(1'-Methoxycarbonylethyl)-N-(pyridin-3"-carbonyl)-2-methyl-6-chlor-anilin bekannt aus DE-OS 25 13 732.

Versuch 1

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten. Das Ergebnis des Versuches zeigt, daß beispielsweise die Verbindungen 1, 2, 3, 4 und 13 bei Anwendung als 0,025%ige Spritzbrühe eine bessere fungizide Wirkung (beispielsweise 100 %) zeigen als der bekannte Wirkstoff A (beispielsweise 70 %).

Versuch 2

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, be-

sprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann. Das Ergebnis des Versuches zeigt, daß beispielsweise die Verbindungen 1, 3 und 13 bei der Anwendung als 0,025-, 0,006- und 0,0015%ige Spritzbrühen eine bessere fungizide Wirkung (beispielsweise 97 %) haben als der bekannte Wirkstoff A (beispielsweise 30 %).

Patentansprüche

1. Thiazolyl- und Isothiazolylcarbonsäureanilid der Formel I

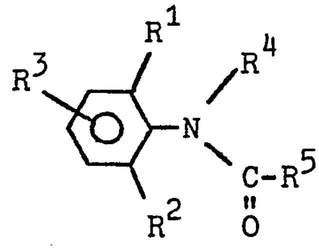

I,

worin
$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_2$-Alkoxy,

$R^4$ $-CH(CH_3)-COOCH_3$ oder 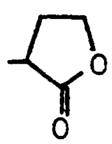 und

$R^5$ einen gegebenenfalls durch Methyl, Chlor, Brom oder Nitro substituierten 3- oder 4- oder 5-Isothiazolyl- oder 2- oder 4- oder 5-Thiazolylrest bedeutet.

2. Thiazolyl- und Isothiazolylcarbonsäureanilid gemäß Anspruch 1, dadurch gekennzeichnet, daß $R^1$ und $R^2$ gleich oder verschieden sind und Methyl, Ethyl, Methoxy, Ethoxy oder Chlor bedeuten, $R^3$ Wasserstoff oder Methyl und $R^5$ 3-, 4- oder 5-Isothiazolyl, 3-Methyl-4-isothiazolyl, 3-Methyl-5-isothiazolyl, 5-Nitro-3-isothiazolyl oder 2-, 4- oder 5-Thiazolyl, 2-Brom-4-thiazolyl, 2-Brom-5-thiazolyl, 5-Nitro-2-thiazolyl, 4-Nitro-2-thiazolyl, 4-Methyl-5-thiazolyl oder 2-Chlor-4-methyl-5-thiazolyl bedeutet.

3. Fungizid, enthaltend eine Verbindung gemäß Anspruch 1.

4. Fungizid, enthaltend eine Verbindung gemäß Anspruch 2.

5. Fungizide Mittel, enthaltend ein Thiazol- oder Isothiazolcarbonsäureanilid gemäß Anspruch 1 und einen inerten Trägerstoff.

6. Verfahren zur Herstellung von Verbindungen gemäß Anspruch 1, dadurch gekennzeichnet, daß man ein substituiertes Anilin der Formel II

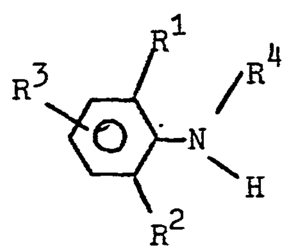

II,

worin
$R^1$, $R^2$, $R^3$ und $R^4$ die in Anspruch 1 angegebenen Bedeutungen haben, mit einem Thiazol- oder Isothiazolcarbonsäurederivat der Formel III

$$R^5-\overset{\overset{O}{\parallel}}{C}-A \qquad III,$$

worin
$R^5$ die im Anspruch 1 angegebene Bedeutung hat und A eine nucleophil verdrängbare Abgangsgruppe bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen -10 und 100°C umsetzt.

7. Verfahren zur Bekämpfung von Pilzen, dadurch gekennzeichnet, daß man eine fungizid wirksame Menge eines Thiazol- oder Isothiazolyl-carbonsäureanilids gemäß Anspruch 1 auf Pilze oder auf durch Pilzbefall bedrohte Flächen, Pflanzen oder Saatgüter einwirken läßt.

Patentansprüche (für Österreich)

1. Fungizid, enthaltend ein Thiazolyl- und Isothiazolylcarbonsäureanilid der Formel I

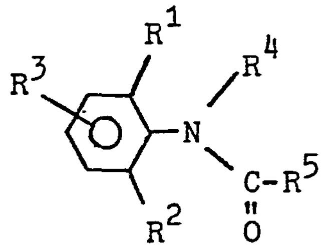

I,

worin

$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_2$-Alkoxy,

$R^4$ 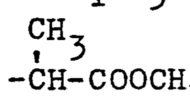 oder 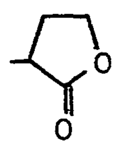 und

$R^5$ einen gegebenenfalls durch Methyl, Chlor, Brom oder Nitro substituierten 3- oder 4- oder 5-Isothiazolyl- oder 2- oder 4- oder 5-Thiazolylrest bedeutet.

2. Fungizid, enthaltend ein Thiazolyl- und Isothiazolylcarbonsäureanilid gemäß Anspruch 1, dadurch gekennzeichnet, daß $R^1$ und $R^2$ gleich oder verschieden sind und Methyl, Ethyl, Methoxy, Ethoxy oder Chlor bedeuten, $R^3$ Wasserstoff oder Methyl und $R^5$ 3-, 4- oder 5-Isothiazolyl, 3-Methyl-4-isothiazolyl, 3-Methyl-5-isothiazolyl, 5-Nitro-3-isothiazolyl oder 2-, 4- oder 5-Thiazolyl, 2-Brom-4-thiazolyl, 2-Brom-5-thiazolyl, 5-Nitro-2-thiazolyl, 4-Nitro-2-thiazolyl, 4-Methyl-5-thiazolyl oder 2-Chlor-4-methyl-5-thiazolyl bedeutet.

3. Fungizide Mittel, enthaltend ein Thiazol- oder Isothiazolcarbonsäureanilid gemäß Anspruch 1 und einen inerten Trägerstoff.

4. Verfahren zur Herstellung von Verbindungen gemäß Anspruch 1, dadurch gekennzeichnet, daß man ein substituiertes Anilin der Formel II

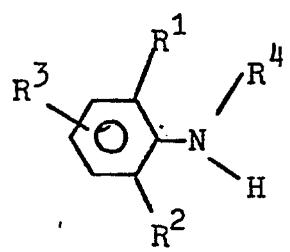

II,

worin
$R^1$, $R^2$, $R^3$ und $R^4$ die in Anspruch 1 angegebenen Bedeutungen haben, mit einem Thiazol- oder Isothiazolcarbonsäurederivat der Formel III

$$R^5-\overset{O}{\overset{\|}{C}}-A \qquad \text{III,}$$

worin
$R^5$ die im Anspruch 1 angegebene Bedeutung hat und A eine nucleophil verdrängbare Abgangsgruppe bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen -10 und 100°C umsetzt.

5. Verfahren zur Bekämpfung von Pilzen, <u>dadurch gekennzeichnet</u>, daß man eine fungizid wirksame Menge eines Thiazol- oder Isothiazolyl-carbonsäureanilids gemäß Anspruch 1 auf Pilze oder auf durch Pilzbefall bedrohte Flächen, Pflanzen oder Saatgüter einwirken läßt.

Europäisches Patentamt

# EUROPÄISCHER RECHERCHENBERICHT

0073973
Nummer der Anmeldung

EP 82 10 7510

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | DE-A-2 513 732 (CIBA-GEIGY) * Ansprüche * | 1-5 | C 07 D 275/02<br>C 07 D 277/56<br>C 07 D 277/58<br>C 07 D 417/12<br>A 01 N 43/78<br>A 01 N 43/80 |
| Y | FR-A-1 546 183 (UNIROYAL INC.) * Zusammenfassung * | 1-5 | |
| Y | US-A-4 144 047 (JOHN E. FRANZ) * Ansprüche; Beispiele 69-72 * | 1-5 | |
| A | DE-A-2 804 299 (CIBA-GEIGY) * Ansprüche * | 1-5 | |
| A | EP-A-0 026 873 (BASF AG) | | |

RECHERCHIERTE SACHGEBIETE (Int. Cl. 3)

C 07 D 275/00
C 07 D 277/00
C 07 D 417/00

Der vorliegende Recherchenbericht wurde für alle Patentansprüche erstellt.

| Recherchenort | Abschlußdatum der Recherche | Prüfer |
|---|---|---|
| DEN HAAG | 09-11-1982 | HENRY J.C. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82